(19) Europäisches Patentamt
European Patent Office
Office européen des brevets

(11) Veröffentlichungsnummer: **0 182 246**
**A1**

(12) **EUROPÄISCHE PATENTANMELDUNG**

(21) Anmeldenummer: **85114339.6**

(22) Anmeldetag: **12.11.85**

(51) Int. Cl.[4]: **C 07 D 233/92**, A 01 N 43/50

(30) Priorität: **23.11.84 DE 3442690**

(43) Veröffentlichungstag der Anmeldung: **28.05.86 Patentblatt 86/22**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Schmierer, Roland, Dr., An der Weinleite 5A, D-8901 Todtenweis (DE)**
Erfinder: **Schulze, Ernst-Friedrich, Dr., Lerchenweg 26, D-6238 Hofheim am Taunus (DE)**
Erfinder: **Bürstell, Helmut, Dr., Am Hohlacker 65, D-6000 Frankfurt am Main 50 (DE)**
Erfinder: **Hacker, Erwin, Dr., Margarethenstrasse 16, D-6230 Hochheim am Main 50 (DE)**

(54) **Salze von 1-Phenyl-imidazol-5-carbonsäuren, ein Verfahren zu ihrer Herstellung und ihre Verwendung als Wachstumsregulatoren.**

(57) Die neuen Salze der im Phenylring substituierten 1-Phenyl-imidazol-5-carbonsäuren der allgemeinen Formel (I)

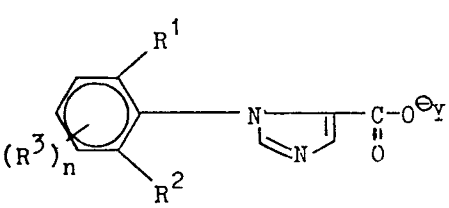

(I)

weisen eine gute das Pflanzenwachstum regulierende Wirkung auf. In der allgemeinen Formel haben die Symbole die folgende Bedeutung:
Y bis zu vierfach durch (subst.) Alkyl, (subst.) Alkenyl, Alkinyl, (subst.) Cycloalkyl, Cycloalkenyl, (subst.) Phenyl substituierte Ammoniumionen, wobei sich das geladene N auch in einen Heterocyclus befinden kann (ausgenommen ist $NH_4^+$); vierfach durch Alkyl, (subst.) Phenyl oder (subst.) Benzyl substituierte Phosphoniumionen; dreifach durch Alkyl, (subst.) Phenyl oder (subst.) Benzyl substituierte Sulfonium- oder Sulfoxoniumionen; das Guanidiniumion oder ein O-Alkyl-isoharnstoff-Kation;
$R^1$, $R^2$ unabhängig voneinander Alkyl;
$R^3$ Alkyl, Alkoxy oder Halogen; und
n 0, 1, 2 oder 3.

In einem Verfahren zur Herstellung dieser Salze wird entweder die freie Carbonsäure oder eines der bekannten Metallsalze (z.B. Na-Salz) mit einer Verbindung Y′ (Y′ = Y minus $H^+$), mit einem Salz mit Y als Kation oder mit einem Hydroxid mit Y als Kation umgesetzt.

Salze von 1-Phenyl-imidazol-5-carbonsäuren, ein Verfahren zu ihrer Herstellung und ihre Verwendung als Wachstumsregulatoren

Die Erfindung betrifft Salze von 1-Phenyl-imidazol-5-carbonsäuren mit einem sich insbesondere von einer organischen Verbindung ableitenden Kation, ein Verfahren zur Herstellung dieser Salze und deren Verwendung als Wachstumsregulatoren.

Im Phenylring substituierte 1-Phenyl-imidazol-5-carbonsäure-Derivate und deren Einsatz als Wachstumsregulatoren sind aus der DE-A 32 17 094 bekannt. Neben der freien Säure und ihren Estern gehören zu den beschriebenen Verbindungen auch die der folgenden allgemeinen Formel,

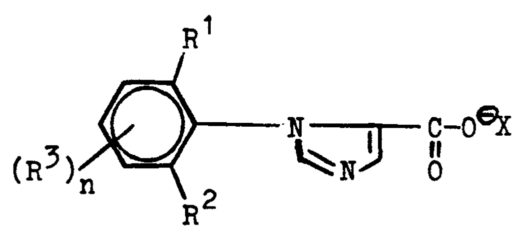

worin die Symbole folgende Bedeutung haben:

| | |
|---|---|
| X | Metallkation oder Ammonium; |
| $R^1$,$R^2$ | unabhängig voneinander $(C_1-C_4)$Alkyl; |
| $R^3$ | $(C_1-C_4)$Alkyl, $(C_1-C_4)$Alkoxy oder Halogen, wobei bei $n > 1$ die Substituenten auch unterschiedlich sein können; und |
| n | 0, 1, 2 oder 3. |

Als Beispiele möglicher Kationen für X werden Metallkationen wie solche des Zn, Cu oder Mn, Alkalikationen wie $Na^+$ und $NH_4^+$ aufgeführt; zu den beanspruchten Verbindungen zählt z.B. das Na-Salz der 1-(2,6-Dimethyl-phenyl)-imidazol-5-carbonsäure.

Einige der bekannten Salze von 1-Phenyl-imidazol-5-carbonsäuren weisen zwar bereits eine ausreichende Wirkung als Wachstumsregulatoren auf, bei ihrem Einsatz müssen jedoch noch verhältnismäßig große Mengen verwendet werden, so daß gelegentlich auch unerwünschte Nebenwirkungen auftreten können.

Aufgabe der vorliegenden Erfindung ist es deshalb, neue wirksamere Salze von 1-Phenyl-imidazol-5-carbonsäuren zu synthetisieren, die auch in geringerer Einsatzmenge als Wachstumsregulatoren angewendet werden können.

Die Erfindung geht aus von den bekannten Salzen der im Phenylring substituierten 1-Phenyl-imidazol-5-carbonsäuren. Die erfindungsgemäßen Verbindungen sind dann dadurch gekennzeichnet, daß in der allgemeinen Formel (I)

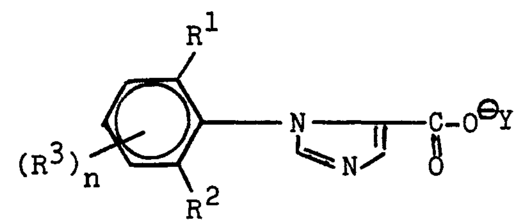

(I)

die Symbole folgende Bedeutung haben:

Y 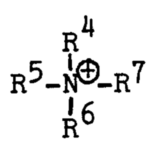 , 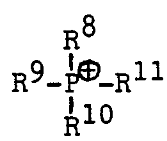 , 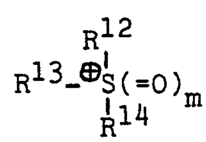 ,

Argininium 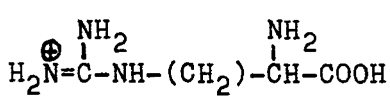 oder das

Guanidiniumion 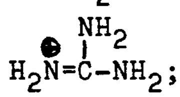;

n 0, 1, 2 oder 3;

m O oder 1;

$R^1$,$R^2$ unabhängig voneinander $(C_1-C_4)$Alkyl;

$R^3$ $(C_1-C_4)$Alkyl, $(C_1-C_4)$Alkoxy oder Halogen, wobei bei $n > 1$ die Substituenten auch unterschiedlich sein können;

$R^4$,$R^5$,$R^6$ unabhängig voneinander H; unsubstituiertes oder bis zu dreifach, vorzugsweise einfach durch Halogen, $(C_1-C_{12})$Alkoxy, vorzugsweise $(C_1-C_6)$-Alkoxy, $[(C_1-C_6)$Alkoxy$]-(C_2-C_4)$alkoxy, Cyclo-$(C_3-C_7)$alkyl, Bicyclo$(C_7-C_{10})$alkyl, Benzyloxy, Halogen-benzyloxy, Methyl-benzyloxy, Benzyloxy-$(C_2-C_4)$alkoxy, Phenyl, Halogen-phenyl, Methylphenyl, Cyano, Hydroxyl, Formyl, $(C_1-C_4)$Alkylcarbonyl, Carboxyl, $(C_1-C_6)$-Alkoxycarbonyl, Carbamoyl, Phosphinyl, Phosphonyl, $(C_1-C_4)$-Alkylamino-phosphonyl, Di$(C_1-C_4)$alkylamino-phosphonyl, Amino, $(C_1-C_6)$Alkylamino, Di$(C_1-C_6)$alkylamino, Amino-$(C_1-C_4)$alkylamino, $(C_1-C_6)$Alkylthio, Phenylthio, Phenoxy, Furyl, Tetrahydrofuryl, Imidazolyl oder Triazolyl substituiertes $(C_1-C_{18})$Alkyl; unsubstituiertes oder durch Halogen oder Phenyl substituiertes $(C_3-C_6)$Alkenyl; $(C_3-C_6)$Alkinyl; unsubstituiertes oder durch $(C_1-C_4)$Alkyl oder Halogen substituiertes Cyclo$(C_3-C_8)$alkyl; Cyclo$(C_5-C_6)$-alkenyl; unsubstituiertes oder bis zu dreifach durch Halogen, Nitro, Cyano, $(C_1-C_4)$Alkyl, $(C_1-C_4)$Alkoxycarbonyl oder $(C_1-C_4)$Alkoxy substituiertes Phenyl;

oder

$R^4$ und $R^5$ bilden gemeinsam mit dem N eine Cyclo$(C_5-C_8)$alkylgruppe, bei der zusätzlich zu dem einen N noch bis zu 2 C durch N, S und/oder O ersetzt sein können und die unsubstituiert oder durch $(C_1-C_4)$Alkyl, Halogen, Phenyl, Benzyl, Oxo, Amino, $(C_1-C_4)$Alkylamino, Di$(C_1-C_4)$-

alkylamino, Amino($C_1$-$C_4$)alkyl, Hydroxyl, Hydroxy-($C_1$-$C_4$)alkyl, ($C_1$-$C_4$)Alkoxy substituiert ist;

$R^7$ H; unsubstituiertes oder durch Phenyl, Halogenphenyl oder Methyl-phenyl substituiertes ($C_1$-$C_{12}$)Alkyl; oder

bei $R^4$, $R^5$ und $R^6$ = H Amino, ($C_1$-$C_4$)Alkylamino, Di-($C_1$-$C_4$)alkylamino, Benzylamino, Anilino, Formylamino, ($C_1$-$C_4$)Alkylcarbonylamino, Benzoylamino, Hydroxyl, ($C_1$-$C_6$)Alkoxy oder $NH-C(NH_2)=Z$ mit Z=O,S oder NH;

ausgenommen sind Verbindungen, bei denen $R^4$, $R^5$, $R^6$ und $R^7$ = H sind;

$R^8$,$R^9$,$R^{10}$,$R^{11}$ unabhängig voneinander ($C_1$-$C_{18}$)Alkyl; unsubstituiertes oder durch Halogen, ($C_1$-$C_4$)Alkyl oder ($C_1$-$C_4$)Alkoxy substituiertes Phenyl; oder unsubstituiertes oder durch Halogen, ($C_1$-$C_4$)Alkyl oder ($C_1$-$C_4$)Alkoxy substituiertes Benzyl; und

$R^{12}$, $R^{13}$,$R^{14}$ unabhängig voneinander ($C_1$-$C_6$)Alkyl, Phenyl, Halogen-phenyl, Methyl-phenyl, Benzyl, Halogen-benzyl oder Methyl-benzyl.

Dabei können die bei der Definition der allgemeinen Formel (I) vorkommenden Alkyl-, Alkenyl- und Alkinylreste sowohl geradkettig als auch verzweigt sein; unter Halogen ist F, Cl, Br oder J, insbesondere F, Cl oder Br zu verstehen. Für den Fall, daß in den Substituenten $R^4$, $R^5$, $R^6$ und $R^7$ weitere basische Stickstoffatome auftreten, ist auch eine mehrfache Salzbildung möglich; d.h. in diesem Fall bedeutet Y nur ein Kationäquivalent, da ein mehrfach geladenes Kation entsprechend der positiven Ladung mehrere Anionen (in der vorliegenden Erfindung 1-Phenyl-imidazol-5-carboxylationen) absättigen kann.

Eine weitere Lösung der gestellten Aufgabe ist auch die Herstellung der erfindungsgemäßen Verbindungen aus den entsprechenden freien Carbonsäuren oder Metallsalzen der allgemeinen Formeln (II) und (III) mit $Met^+$ in der Bedeutung eines einfach positiv geladenen Metallkations bzw. eines Kationäquivalents bei einem mehrfach positiv geladenen Metallkation, insbesondere bedeutet $Met^+ = Na^+$,

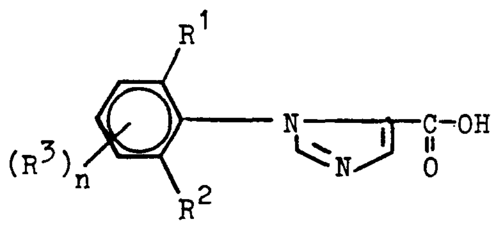

(II)

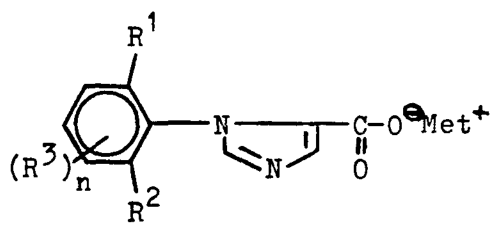

(III)

a) durch Umsetzung von (II) mit den Aminoverbindungen Y' ($Y'=Y-H^+$) oder mit Salzen mit Y als Kation, wobei Y

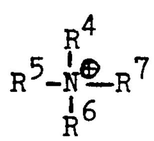

,

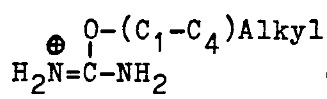

oder

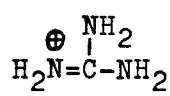

bedeutet und mindestens einer der Reste $R^4$ bis $R^7$=H ist, oder

b) durch Umsetzung von (II) mit den Verbindungen $YHal^-$ oder $YOH^-$ oder von (III) mit den Verbindungen $YHal^-$, wobei Y

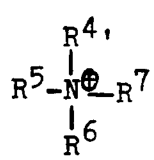

,

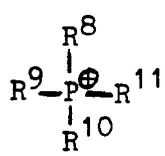

oder

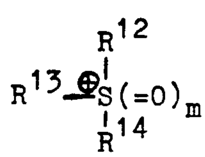

,

und alle Reste $R^4$ bis $R^7 \neq H$ sind.

In der Verfahrensvariante a) ist bei Einsatz der Aminverbindungen Y' (d.h. Y'=Y minus $H^+$) die Verwendung äquimolarer Mengen der Komponenten vorteilhaft. Die Salzbildung verläuft im allgemeinen problemlos, sie kann in An- oder Abwesenheit von Lösemitteln wie Wasser, Alkoholen, Aceton, Halogenkohlenwasserstoffen, Ethern, Toluol, Xylol oder aliphatischen oder cycloaliphatischen Kohlenwasserstoffen durchgeführt werden. Vorteilhaft wird das Lösemittel für die Reaktion so ausgewählt, daß sich die Säure schlecht, das gebildete Salz jedoch gut löst. Der Reaktionsverlauf und der Umsatz der Komponenten lassen sich dann leicht am Auflösen des Niederschlages beobachten. Solche geeigneten Lösemittel sind zum Beispiel Wasser, Ethanol, Methanol, Chloroform oder Methylenchlorid. Die Reaktionstemperatur der Umsetzung ist unkritisch; sie liegt im allgemeinen zwischen -20°C und +100°C, insbesondere jedoch zwischen 0°C und +50°C, wobei die Salzbildung häufig schwach exotherm verläuft. Die erfindungsgemäßen Verbindungen fallen, wenn ohne Lösemittel gearbeitet wird, direkt an; wird mit einem Lösemittel gearbeitet, empfiehlt es sich, dieses zur Isolierung der Produkte abzudestillieren. Zur Kristallisation der Rohprodukte kann gegebenenfalls mit unpolaren Lösemitteln verrieben werden (z.B. Ether, Hexan). Die Salze werden dann vom Lösemittel abgesaugt und fallen in sehr guter Ausbeute und Reinheit an.

Die Aminoverbindungen Y' können auch, wenn dies beispielsweise auf Grund ihrer Handhabbarkeit oder Stabilität nötig ist, als Salze (mit Y als Kation) eingesetzt und erst in Gegenwart der Carbonsäure der allgemeinen Formel (II) in die freien Basen Y' überführt werden; als Beispiele sollen hier Hydroxylammoniumchlorid, Hydrochloride von Aminosäureestern oder Hydrochloride oder Sulfate von Aminoharnstoff- oder Thioharnstoff genannt werden. Hierzu kann beispielsweise die Zugabe von Propylenoxid oder die Neutralisation

der Hydrohalogenide mit stöchiometrischen Mengen an anorganischen Basen wie Alkali- und Erdalkalihydroxiden, -carbonaten oder -hydrogencarbonaten dienen. Die dabei als Nebenprodukte entstehenden anorganischen Salze können nach der Umsetzung aus dem Reaktionsgemisch leicht durch Auflösen der erfindungsgemäß hergestellten Verbindungen in geeigneten Lösemitteln wie Methylenchlorid oder Aceton und Absaugen der darin unlöslichen anorganischen Salze abgetrennt werden. Die Weiterverarbeitung der erfindungsgemäßen Verbindungen erfolgt dann wie vorstehend beschrieben.

Sind mehrere basische Aminogruppen vorhanden, so können auf vergleichbare Weise durch Einsatz der entsprechenden ein-, zwei- oder dreimolaren Menge an Carbonsäure der allgemeinen Formel (II) die entsprechenden Mono-, Di- bzw. Trisalze hergestellt werden. Als Beispiele für Aminverbindungen Y' mit mehreren basischen Stellen können Hydrazin, Ethylendiamin, Propylendiamin, Pyrazin oder Diethylentriamin genannt werden.

In der Verfahrensvariante b), in der die Carbonsäuren der allgemeinen Formel (II) mit $YHal^-$ (Ammonium-, Phosphonium-, Sulfonium- oder Sulfoxoniumhalogenide) unter Abtrennung von HHal (Halogenwasserstoff) eingesetzt werden, dient zur Abspaltung des HHal's die Zugabe von unter den Reaktionsbedingungen irreversibel mit HHal reagierende Substanzen wie Silberoxid oder Propylenoxid. Die Umsetzung wird bevorzugt in Wasser, Methanol oder Ethanol durchgeführt. Die Isolierung der Produkte erfolgt nach Abtrennen der Nebenprodukte (z.B. Silberhalogenide durch Filtration, Halogenhydrine durch Extraktion) durch Eindampfen des Reaktionsgemisches.

In der Verfahrensvariante b), in der Carbonsäure der allgemeinen Formel (II) mit $YOH^-$ (Ammonium-, Phosphonium-,

Sulfonium- oder Sulfoxoniumhydroxide) unter Abtrennung von $H_2O$ umgesetzt werden, können dem Reaktionsgemisch wasserentziehende Mittel wie Molekularsiebe zugesetzt werden, oder das Wasser wird vorteilhaft durch azeotrope Destillation unter Verwendung eines geeigneten Lösemittels wie Toluol, Benzol oder Chloroform entfernt.

In der Verfahrensvariante b), in der die Salze von Carbonsäuren entsprechend der allgemeinen Formel (III) mit $YHal^-$ unter Abtrennung des $Met^+Hal^-$ (Metallhalogenide) umgesetzt werden, kann man entweder das wasserfreie Metallcarboxylat der allgemeinen Formel (III) mit dem Halogenid, z.B. in einem organischen Lösemittel wie Toluol, Xylol, Chloroform, Aceton oder Ethanol, bei Temperaturen zwischen 20°C und dem Siedepunkt des Lösemittels zur Reaktion bringen, wobei das Metallhalogenid nach beendigter Reaktion abgesaugt und das Produkt durch Eindampfen des Lösemittels isoliert werden kann, oder man setzt die Carbonsäure der allgemeinen Formel (II) zunächst in Wasser mit der äquivalenten Menge einer anorganischen Base wie Alkalimetallhydroxiden oder -carbonaten zu den Metallcarboxylaten der allgemeinen Formel (III) um. Zu dieser wäßrigen Metallsalzlösung gibt man dann das Halogenid zu und erhitzt auf 50°C bis 100°C. Zur Isolierung des gewünschten Produkts wird von den Lösemitteln abdestilliert und das organische Salz durch Lösen in einem organischen Lösemittel von dem anorganischen Salz abgetrennt.

Mit den erfindungsgemäßen Verbindungen sind typische wachstumsregulierende Effekte erzielbar, die - verglichen den aus der DE-A 32 17 094 bekannten Verbindungen, auch mit den

bereits von dort bekannten Salzen - bereits bei niedrigeren Dosierungen einsetzen (z.B. bei einem Vergleich mit der strukturell am ehesten vergleichbaren Ammoniumverbindung mit $NH_4^+$ als Kation). Sie greifen regulierend in den pflanzeneigenen Stoffwechsel ein und können damit zur gezielten Beeinflussung von Pflanzeninhaltsstoffen sowie zur Ernteerleichterung wie zum Auslösen von Desikkation und Wuchsstauchung eingesetzt werden. Des weiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Rasen sowie ihre Fähigkeiten, den Gehalt an erwünschten Inhaltsstoffen wie Kohlehydraten (z.B. in Zuckerrohr- oder Hirsekulturen) und Protein bei Nutzpflanzen zu erhöhen. Schließlich zeigen die Verbindungen eine sehr gute Verbesserung der Fruchtabszission, insbesondere bei Zitrusfrüchten.

Eine weitere Lösung der gestellten Aufgabe sind auch das Pflanzenwachstum regulierende Mittel, die sich durch einen wirksamen Gehalt mindestens einer Verbindung der allgemeinen Formel (I) auszeichnen. Die Aufwandmenge der erfindungsgemäßen Verbindungen beträgt im allgemeinen 0,02 bis 1,5 kg Wirksubstanz pro ha, vorzugsweise 0,05 bis 1 kg/ha.

Die erfindungsgemäßen Verbindungen lassen sich bei ihrem praktischen Einsatz gegebenenfalls auch vorteilhaft mit bekannten Wachstumsregulatoren kombinieren. Die das Pflanzenwachstum regulierenden Mittel können dann erfindungsgemäß neben mindestens einer Verbindung der allgemeinen

Formel (I) gegebenenfalls auch noch zusätzlich weitere Wirkstoffe aus chemisch unterschiedlichen Gruppen von Wachstumsregulatoren enthalten. Die bevorzugten kombinierten Mittel enthalten mindestens eine Verbindung der allgemeinen Formel (I) in Kombination mit einer Verbindung der Formel (IV),

$$R-CH_2-CH_2-N^{\oplus}(CH_3)_3Cl \qquad (IV)$$

worin R= OH oder Cl bedeutet,

oder mit einer Verbindung der Formel (V),

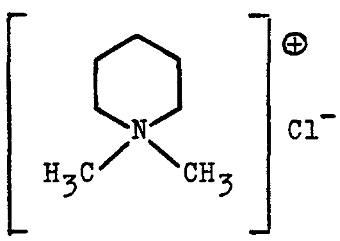

(V)

oder mit einer Verbindung der Formel (VI),

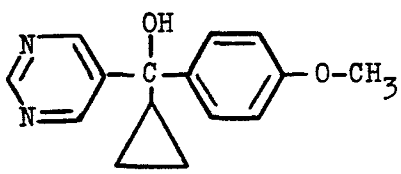

(VI)

oder mit einer Verbindung der Formel (VII),

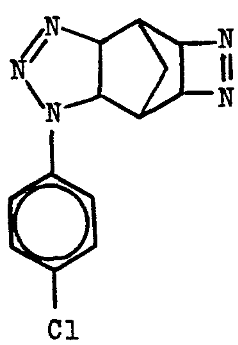

(VII)

oder mit einer Verbindung der Formel (VIII),

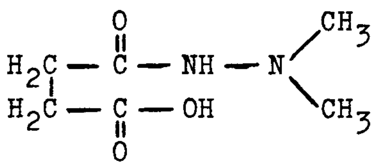 (VIII)

oder mit einer Verbindung der Formel (IX),

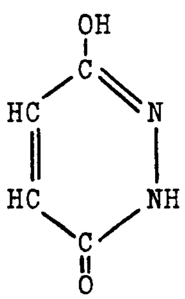 (IX)

oder mit einer Verbindung der Formel (X),

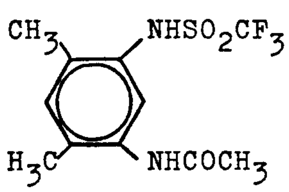 (X)

oder mit einer Verbindung der Formel (XI),

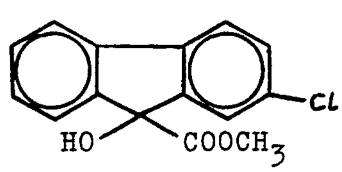 (XI)

oder mit einer Verbindung der Formel (XII)

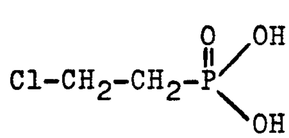 (XII).

Die Verbindungen der Formeln (IV) bis (XII) sind bekannte Handelsprodukte. Die Verbindung der Formel (IV) mit R= Cl [Verbindung (IVa)] besitzt den common name Chlormequat. Die Verbindung der Formel (V) ist unter dem Namen Mepiquatchlorid, die Verbindung der Formel (VI) als Ancymidol, die Verbindung der Formel (VII) als Tetcyclacis, und die Verbindung der Formel (X) als Mefluidid und die Verbindung der Formel (XII) als Ethephon bekannt. Die wachstumsregulatorischen Wirkungen der Verbindungen der Formeln (IV) bis (XII) sind beschrieben in Plant Growth Regulator Handbook of the Plant Growth Regulator Working Group 2d. Ed. 1981.

Anstelle der Verbindungen der Formeln (VI) und (V) können prinzipiell auch vergleichbare Salze, die anstelle des Chloridions ein anderes übliches Anion wie Bromid, Nitrat oder 1/2 Sulfat enthalten, eingesetzt werden.

Bei der Kombination der Verbindungen der Formel (I) mit den Verbindungen der Formeln (IV) bis (XII) zeigen sich überraschenderweise auffallende synergistische Wirkungen. So lassen sich diese Kombinationen in noch geringeren Dosierungen einsetzen, als von der Wirkung der Einzelkomponenten zu erwarten war, um die gewünschten Effekte zu erzielen. Mit den Kombinationen lassen sich auch Wildwuchserscheinungen vermindern, so daß die Kombinationen auch in der Landschaftspflege eingesetzt werden können. Desweiteren eignen sich die Kombinationen hervorragend zur generellen Steuerung und Hemmung von unerwünschtem vegativen Wachstum, wie Seitentriebsbildung, ohne die Pflanzen abzutöten. Die Verbindungen der Formel (I) können auch vorteilhaft mit zwei verschiedenen Verbindungen der Formeln (IV) bis (XII) kombiniert werden.

Die Mischungsverhältnisse der Komponenten der allgemeinen Formel (I) zu den Verbindungen der Formeln (IV) bis (XII) können innerhalb weiter Grenzen, etwa zwischen 250 : 1 bis 1 : 10, schwanken. Die Wahl des Mischungsverhältnisses ist abhängig von der Art der Mischungspartner, vom Entwicklungsstadium der Pflanzen als auch vom Grad der gewünschten wachstumsregulatorischen Wirkung. Vorzugsweise werden Mischungsverhältnisse von 10 : 1 bis 1 : 10 gewählt.

Die Aufwandmenge der Verbindungen der Formel (I) in den Wirkstoffmischungen liegt im allgemeinen zwischen 0,05 und 1 kg Wirkstoff pro ha, die Aufwandmengen der Verbindungen der Formel (IV) bis (XII) variieren zwischen 0,01 und 5 kg Wirkstoff/ha. Die Kombinationen können sowohl als Mischformulierungen der Komponenten - z.B. benetzbare Pulver oder Emulsionskonzentrate - vorliegen, die dann in üblicher Weise mit Wasser verdünnt zur Anwendung gebracht werden, oder als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

Die erfindungsgemäßen Mittel können neben den vorstehend genannten Wirkstoffen, d.h. insbesondere mindestens einer Verbindung der allgemeinen Formel (I) und gegebenenfalls einem anderen Wachstumsregulator, auch noch natürliche oder pflanzliche Hormone wie Auxine oder Cytokinine enthalten.

Die erfindungsgemäßen Verbindungen der allgemeinen Formel (I) können, gegebenenfalls im Gemisch mit weiteren Wirkkomponenten, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs- oder Inertstoff noch Netzmittel wie polyoxethylierte Fettalkohole, Alkyl- oder Alkylphenylsulfonate und/oder Dispergierhilfsmittel wie ligninsulfonsaures Natrium, 2,2'-dinaphthylmethan-6,6'-disulfonsaures Natrium, dibutylnapthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des (der) Wirkstoffe(s) in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des (der) Wirkstoffe(s) mit feinverteilten, festen Stoffen, z.B. Talkum, natürlichen Tonen wie Kaolin, Bentonit, Pyrophillit oder Diatomeenerde erhalten. Granulate können entweder durch Verdüsen des (der) Wirkstoffe(s) auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstofformulierungen gegebenenfalls die jeweils üblichen Haft-, Netz-, Dispergier-, Emulgier-, Penetrations-, Lösemittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen wie Insektiziden, Akariziden, Herbiziden, Düngemitteln oder Fungiziden sind gegebenenfalls möglich.

In den nachfolgenden Beispielen verhalten sich Gew.-Teile (GT) zu Vol.-Teilen (VT) wie kg zu $dm^3$ (l) und %-Angaben beziehen sich - falls nichts anderes angegeben ist - auf das Gewicht.

## FORMULIERUNGSBEISPIELE

### Beispiel 1

Ein Stäubemittel wird erhalten, indem man a) 10 GT Wirkstoff(e) und 90 GT Talkum oder einem anderen Inertstoff mischt und in einer Schlagmühle zerkleinert, oder indem man b) 60 GT Wirkstoff, 35 GT Talkum und 5 GT Haftmittel (z.B. ein Polysaccharid wie (R)Rhodopol der Rhone-Poulenc S.A.) in der gleichen Weise homogenisiert.

### Beispiel 2

Ein in Wasser leicht dispergierbares, benetzbares Pulver wird erhalten, indem man 25 GT Wirkstoff(e), 64 GT kaolinhaltigen Quarz als Inertstoff, 10 GT ligninsulfonsaures Kalium und 1 GT oleoylmethyltaurinsaures Natrium als Netz- und Dispergierhilfsmittel mischt und in einer Stiftmühle mahlt. Eine Formulierung mit 5 % Wirkstoffgehalt kann wie folgt zusammengesetzt sein: 5 % Wirkstoff(e), 6 % eines sulfonierten Napthalinformaldehydkondensats (z.B. (R)Dispersogen A der HOECHST AG), 2 % eines Na-Salzes einer Alkylnapthalinsulfonsäure (z.B. (R)Leonil DB der HOECHST AG), 5 % eines Gemisches aus Polypropylenglykol und $SiO_2$ (z.B. (R)Acrotin 341 der HOECHST AG), 25 % eines $SiO_2$ (z.B. (R)Sipernat der Degussa AG) und 57 % Kaolin Typ 1777.

### Beispiel 3

Ein in Wasser leicht dispergierbares Dispersionskonzentrat wird erhalten indem man 20 GT Wirkstoff(e) mit 6 GT eines Alkylphenolpolyglykolethers (z.B. (R)Triton X 207 von Rohm and Haas Co.), 3 GT Isotridecanolpolyglykolether (8 Ethylenoxid-Einheiten) und 71 GT paraffinischem Mineralöl (Siedebereich ca. 255 bis über 377°C) mischt und in einer Reibkugelmühle auf eine Feinheit von unter 5 µm vermahlt.

Beispiel 4

Ein emulgierbares Konzentrat wird erhalten aus 15 GT Wirkstoff(e), 75 GT Cyclohexanon als Lösemittel und 10 GT oxethyliertem Nonylphenol (10 Ethylenoxid-Einheiten) als Emulgator.

CHEMISCHE BEISPIELE

Beispiel 1

Methylammonium-1-(2,6-diethylphenyl)-imidazol-5-carboxylat

Zu 15 GT (0,061 mol) der 1-(2,6-Diethylphenyl)-imidazol-5-carbonsäure in 50 VT Methylenchlorid tropft man 5,7 GT (0,073 mol) einer 40%igen wäßrigen Methylaminlösung. Nach 2 h wird im Vakuum eingedampft, der Rückstand mit 100 VT Diethylether verrührt und abgesaugt. Man erhält 15,7 GT (93 % d. Theorie) des Methylammonium-1-(2,6-diethylphenyl)-imidazol-5-carboxylats, einem farblosen Feststoff mit einem Schmelzpunkt (Fp) von 110 bis 113°C.

Beispiel 2

Ethoxycarbonylmethylammonium-1-(2,6-diethylphenyl)-imidazol-5-carboxylat

Zu 25 GT (0,10 mol) der 1-(2,6-Diethylphenyl)-imidazol-5-carbonsäure, 15,7 GT (0,11 mol) des Glycinethylester-Hydrochlorids und 50 VT Methylenchlorid tropft man 14,9 GT (0,25 mol) Propylenoxid. Nach 2 h Erhitzen unter Rückfluß ist eine klare Lösung entstanden. Man läßt abkühlen und dampft ein. Das verbleibende Öl wird zweimal mit je 100 VT Diethylether digeriert und getrocknet. Man erhält 22,8 GT (63 % d. Theorie) des Ethoxycarbonylmethylammonium-1-(2,6-diethylphenyl)-imidazol-5-carboxylats, einem farblosen hochviskosen Öl; die Identifizierung erfolgt $^1$H-NMR-spektroskopisch.

Beispiel 3

(2-Hydroxyethyl-trimethylammonium)-1-(2,6-diethylphenyl)-imidazol-5-carboxylat

25 GT (0,10 mol) der 1-(2,6-Diethylphenyl)-imidazol-5-carbonsäure und 27,0 GT (0,11 mol) einer 50%igen wäßrigen Cholinlösung werden in 100 VT Toluol bis zur vollständigen Entwässerung am Wasserabscheider erhitzt. Man läßt erkalten und dampft im Vakuum ein. Man erhält 34,5 GT (97 % d. Theorie) des (2-Hydroxyethyl-trimethylammonium)-1-(2,6-diethylphenyl)-imidazol-5-carboxylats, einem hochviskosen Öl; die Identifizierung erfolgt $^1$H-NMR-spektroskopisch.

Beispiel 4

Tetraethylammonium-1-(2,6-diethylphenyl)-imidazol-5-carboxylat

15 GT (0,061 mol) der 1-(2,6-Diethylphenyl)-imidazol-5-carbonsäure werden mit 14,0 GT (0,067 mol) des Tetraethylammoniumbromids und 4,5 GT (0,078 mol) an Propylenoxid während 24 h bei Raumtemperatur in 50 VT Wasser gerührt. Die nach dieser Zeit homogene Phase wird zweimal mit Diethylether gewaschen und die wäßrige Phase eingedampft. Man erhält 20,4 GT (89 % d. Theorie) des Tetraethylammonium-1-(2,6-diethylphenyl)-imidazol-5-carboxylats, einem viskosen Öl; die Identifizierung erfolgt $^1$H-NMR-spektroskopisch.

Beispiel 5

(2-Chlorethyl-trimethylammonium)-1-(2,6-diethylphenyl)-imidazol-5-carboxylat

15 GT (0,061 mol) der 1-(2,6-Diethylphenyl)-imidazol-5-carbonsäure werden mit 10,2 GT (0,064 mol) Cholindichlorid und 5,5 GT (0,065 mol) Natriumhydrogencarbonat in 50 VT Wasser während 8 h bei 50°C gerührt. Nach dieser Zeit bildet sich eine homogene Lösung; man dampft ein, nimmt in 200 VT Methylenchlorid auf, filtriert die anorganischen Salze ab und dampft ein. Man erhält 19,6 GT (87 % d. Theorie) des (2-Chlorethyl-trimethylammonium)-1-(2,6-diethylphenyl)-imidazol-5-carboxylats, einem viskosen Öl; die Identifizierung erfolgt $^{1}H$-NMR-spektroskopisch.

In Anlehnung an die vorstehenden Beispiele werden auch die in der Tabelle 1 aufgeführten Substanzen hergestellt.

Tabelle 1 Verbindungen der Formel (I)

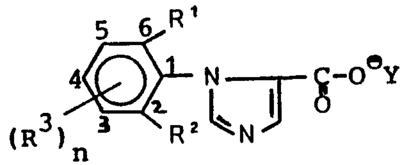

(I)

| Bsp.-Nr. | $R^1$ | $R^2$ | $R^3/n$ | Y | hergestellt nach Beispiel | Fp. (°C) |
|---|---|---|---|---|---|---|
| 6 | $CH_3$ | $CH_3$ | -/O | $H_2\overset{\oplus}{N}-(CH(CH_3)_2)_2$ | 1 | |
| 7 | " | " | " | $H_2\overset{\oplus}{N}(-CH(CH_3)_2)$- 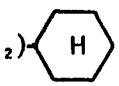 | 1 | |
| 8 | " | " | $4-CH_3/1$ | $(C_4H_9)_3\overset{\oplus}{P}-C_{18}H_{37}$ | 4 | |
| 9 | " | " | " | $(C_4H_9)_3\overset{\oplus}{P}-C_{16}H_{33}$ | 4 | |
| 10 | " | $C_2H_5$ | $5-CH_3/1$ | $H_3\overset{\oplus}{N}-CH_2$- 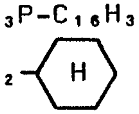 | 1 | |
| 11 | $C_2H_5$ | " | -/O | $H_2\overset{\oplus}{N}(-CH_3)_2$ | 1 | 78-80 |
| 12 | " | " | " | $H\overset{\oplus}{N}(-CH_3)_3$ | 1 | Harz |
| 13 | " | " | " | $H_3\overset{\oplus}{N}-C_2H_5$ | 1 | 72-8 |
| 14 | " | " | " | $H\overset{\oplus}{N}(-C_2H_5)_3$ | 1 | 130-5 |
| 15 | " | " | " | $H_3\overset{\oplus}{N}-CH(CH_3)_2$ | 1 | 144-5 |
| 16 | " | " | " | $H_3\overset{\oplus}{N}-C_4H_9$ | 1 | 149-56 |

Fortsetzung Tabelle 1

| Bsp.-Nr. | $R^1$ | $R^2$ | $R^3/n$ | Y | hergestellt nach Beispiel | Fp. (°C) |
|---|---|---|---|---|---|---|
| 17 | $C_2H_5$ | $C_2H_5$ | -/O | $H_2\overset{\oplus}{N}(-C_4H_9)_2$ | 1 | 147-54 |
| 18 | " | " | " | $H\overset{\oplus}{N}(-C_4H_9)_3$ | 1 | 108-14 |
| 19 | " | " | " | $H_3\overset{\oplus}{N}(-CH_2)_2-CH(CH_3)_2$ | 1 | 135-42 |
| 20 | " | " | " | $H_3\overset{\oplus}{N}-n-C_{12}H_{25}$ | 1 | |
| 21 | " | " | " | $H_3\overset{\oplus}{N}-n-C_{18}H_{37}$ | 1 | Wachs |
| 22 | " | " | " | $H_2\overset{\oplus}{N}-n-(C_{18}H_{37})_2$ | 1 | " |
| 23 | " | " | " | $H_3\overset{\oplus}{N}-CH_2-CH_2-OCH_3$ | 1 | 121-3 |
| 24 | " | " | " | $H_3\overset{\oplus}{N}-CH_2-CH(OCH_3)_2$ | 1 | 114-6 |
| 25 | " | " | " | $H_3\overset{\oplus}{N}(-CH_2)_3-O-CH_3$ | 1 | 153-6 |
| 26 | " | " | " | $H_3\overset{\oplus}{N}(-CH_2)_3-O-CH(CH_3)_2$ | 1 | 133-4 |
| 27 | " | " | " | $H_3\overset{\oplus}{N}-CH_2$ 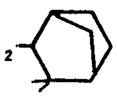 | 1 | 160-1 |
| 28 | " | " | " | $H_3\overset{\oplus}{N}-CH_2-CH_2$ 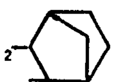 | 1 | 206-10 |
| 29 | " | " | " | $H_3\overset{\oplus}{N}-CH_2$ 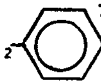 | 1 | 171-4 |

...

Fortsetzung Tabelle 1

| Bsp.-Nr. | $R^1$ | $R^2$ | $R^3/n$ | Y | hergestellt nach Beispiel | Fp. (°C) |
|---|---|---|---|---|---|---|
| 30 | $C_2H_5$ | $C_2H_5$ | -/O | $H_3\overset{\oplus}{N}-CH_2-CN$ | 2 | |
| 31 | " | " | " | $H_3\overset{\oplus}{N}-CH_2-CH_2-OH$ | 1 | 108-16 |
| 32 | " | " | " | $H_2\overset{\oplus}{N}(-CH_2-CH_2-OH)_2$ | 1 | 141-9 |
| 33 | " | " | " | $H\overset{\oplus}{N}(-CH_2-CH_2-OH)_3$ | 1 | 83-8 |
| 34 | " | " | " | $(CH_3-)_2\overset{\oplus}{N}H-CH_2-CH_2-OH$ | 1 | 119-21 |
| 35 | " | " | " | $H_3\overset{\oplus}{N}-CH_2-CH(OH)-CH_3$ | 1 | 102-14 |
| 36 | " | " | " | $H_3\overset{\oplus}{N}-C(CH_3)_2-CH(OH)-CH_3$ | 1 | 80-4 |
| 37 | " | " | " | $(CH_3-)_2\overset{\oplus}{N}H(-CH_2)_3-OH$ | 1 | 134-8 |
| 38 | " | " | " | $H_3\overset{\oplus}{N}-CH(CH_3)-COOCH_3$ | 2 | Oel |
| 39 | " | " | " | $H_3\overset{\oplus}{N}-CH_2-COOCH_3$ | 2 | 140-54(Z) |
| 40 | " | " | " | $H_3\overset{\oplus}{N}-\underset{CONH_2}{\underset{\mid}{C}}(CH_3)-CH(CH_3)_2$ | 1 | |
| 41 | " | " | " | $H_3\overset{\oplus}{N}-CH_2-CH_2-NH_2$ | 1 | |
| 42 | " | " | " | $1/2\cdot(H_3\overset{\oplus}{N}-CH_2-CH_2-\overset{\oplus}{N}H_3)$ | 1 | 185-95 ... |

Fortsetzung Tabelle 1

| Bsp.-Nr. | $R^1$ | $R^2$ | $R^3$/n | Y | hergestellt nach Beispiel | Fp. (°C) |
|---|---|---|---|---|---|---|
| 43 | $C_2H_5$ | $C_2H_5$ | -/O | $H_2\overset{\oplus}{N}(-CH_2-CH_2-NH_2)_2$ | 1 | |
| 44 | " | " | " | $(CH_3-)_2\overset{\oplus}{N}H-CH_2-CH_2-N(CH_3)_2$ | 1 | |
| 45 | " | " | " | $H_3\overset{\oplus}{N}-CH_2-CH=CH_2$ | 1 | |
| 46 | " | " | " | $H_2\overset{\oplus}{N}(-CH_2-CH=CH_2)_2$ | 1 | |
| 47 | " | " | " | 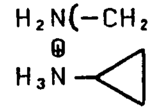 | 1 | |
| 48 | " | " | " | 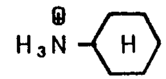 | 1 | 172-4 |
| 49 | " | " | " | 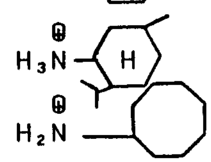 | 1 | Harz |
| 50 | " | " | " | | 1 | |
| 51 | " | " | " | 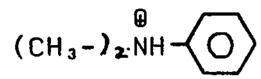 | 1 | |
| 52 | " | " | " | 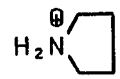 | 1 | 173-6 |
| 53 | " | " | " | 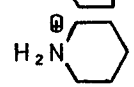 | 1 | 174-7 |

Fortsetzung Tabelle 1

| Bsp.-Nr. | $R^1$ | $R^2$ | $R^3$/n | Y | hergestellt nach Beispiel | Fp. (°C) |
|---|---|---|---|---|---|---|
| 54 | $C_2H_5$ | $C_2H_5$ | -/O | 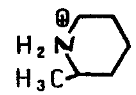 | 1 | 187-90 |
| 55 | " | " | " | 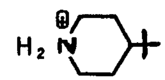 | 1 | Harz |
| 56 | " | " | " | $HO-CH_2-CH_2-$ 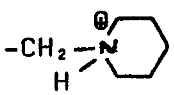 | 1 | Harz |
| 57 | " | " | " | 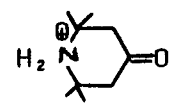 | 1 | 153 (Zers.) |
| 58 | " | " | " | 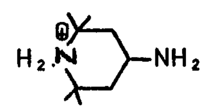 | 1 | 204-7 |
| 59 | " | " | " | 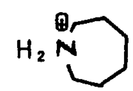 | 1 | 100-2 |
| 60 | " | " | " | 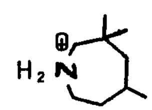 | 1 | 118-26 (Z) |

Fortsetzung Tabelle 1

| Bsp.-Nr. | $R^1$ | $R^2$ | $R^3/n$ | Y | hergestellt nach Beispiel | Fp. (°C) |
|---|---|---|---|---|---|---|
| 61 | $C_2H_5$ | $C_2H_5$ | -/O | 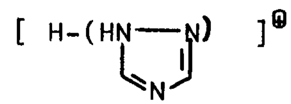 | 1 | |
| 62 | " | " | " | 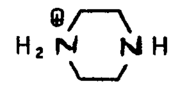 | 1 | |
| 63 | " | " | " | 1/2 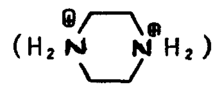 | 1 | |
| 64 | " | " | " | 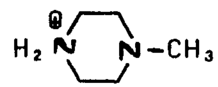 | 1 | 102-7 |
| 65 | " | " | " | 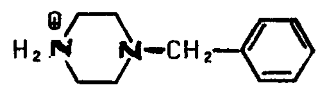 | 1 | |
| 66 | " | " | " | 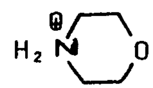 | 1 | Oel |
| 67 | " | " | " | 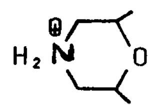 | 1 | 101-7 |

Fortsetzung Tabelle 1

| Bsp.-Nr. | $R^1$ | $R^2$ | $R^3/n$ | Y | hergestellt nach Beispiel | Fp. (°C) |
|---|---|---|---|---|---|---|
| 68 | $C_2H_5$ | $C_2H_5$ | -/O | $HO-CH_2-CH_2-$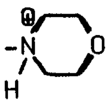 | 1 | Oel |
| 69 | " | " | " | $\overset{\oplus}{N}(-C_4H_9)_4$ | 4 | Harz |
| 70 | " | " | " | 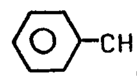$-CH_2-\overset{\oplus}{N}(-C_2H_5)_3$ | 4 | Oel |
| 71 | " | " | " | $(CH_3-)_3\overset{\oplus}{N}-C_8H_{17}$ | 4 | |
| 72 | " | " | " | $(C_2H_5-)_3\overset{\oplus}{N}-C_{12}H_{25}$ | 4 | |
| 73 | " | " | " | $H_3\overset{\oplus}{N}-NH_2$ | 1 | |
| 74 | " | " | " | $1/2\cdot(H_3\overset{\oplus}{N}-\overset{\oplus}{N}H_3)$ | 1 | 135-40 |
| 75 | " | " | " | $H_3\overset{\oplus}{N}-NHCHO$ | 1 | 123-9 |
| 76 | " | " | " | $H_3\overset{\oplus}{N}-NH-Phenyl$ | 1 | |
| 77 | " | " | " | $H_3\overset{\oplus}{N}-OH$ | 2 | Oel |
| 78 | " | " | " | $H_3\overset{\oplus}{N}-OCH_3$ | 2 | " |

Fortsetzung Tabelle 1

| Bsp.-Nr. | $R^1$ | $R^2$ | $R^3/n$ | Y | hergestellt nach Beispiel | Fp. (°C) |
|---|---|---|---|---|---|---|
| 79 | $C_2H_5$ | $C_2H_5$ | -/0 | $H_3\overset{\oplus}{N}-NH-(C=NH)NH_2$ | 2 | 100-2 |
| 80 | " | " | " | $H_3\overset{\oplus}{N}-NH-CS-NH_2$ | 2 | |
| 81 | " | " | " | $[H-(H_2N-\overset{NH_2}{\overset{\Vert}{C}}-NH_2)]^{\oplus}$ | 2 | 193-4 |
| 82 | " | " | " | $CH_3-\overset{\oplus}{P}(-C_4H_9)_3$ | 4 | Harz |
| 83 | " | " | " | $\overset{\oplus}{P}(-C_4H_9)_4$ | 4 | |
| 84 | " | " | " | $\overset{\oplus}{P}(-C_8H_{17})_4$ | 4 | |
| 85 | " | " | " | $CH_3-\overset{\oplus}{P}(-Phenyl)_3$ | 4 | |
| 86 | " | " | " | $\overset{\oplus}{P}(-Phenyl)_4$ | 4 | |
| 87 | " | " | " | $\overset{\oplus}{S}(-CH_3)_3$ | 4,5 | Harz |
| 88 | " | " | " | $O-\overset{\oplus}{S}(-CH_3)_3$ | 4 | 133-43 |
| 89 | " | " | " | $\overset{\oplus}{S}(-CH_3)_2-CH_2-Phenyl$ | 4 | |
| 90 | " | " | 4- Br/1 | 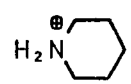 | 1 | 135-8 |

Fortsetzung Tabelle 1

| Bsp.-Nr. | $R^1$ | $R^2$ | $R^3$ /n | Y | hergestellt nach Beispiel | Fp. (°C) |
|---|---|---|---|---|---|---|
| 91 | $C_2H_5$ | $C_2H_5$ | 4-Br /1 | 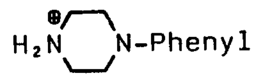 | 1 | |
| 92 | " | " | " | $H_3\overset{\oplus}{N}-N(CH_3)_2$ | 1 | |
| 93 | " | $CH(CH_3)_2$ | —/O | $H_2\overset{\oplus}{N}(-C_2H_5)_2$ | 1 | |
| 94 | " | " | " | $H_3\overset{\oplus}{N}-n-C_3H_7$ | 1 | |
| 95 | " | " | " | $H_2\overset{\oplus}{N}-n-(C_{18}H_{37})_2$ | 1 | Wachs |
| 96 | " | " | " | $H_3\overset{\oplus}{N}(-CH_2)_2-O-(CH_2)_2-OCH_3$ | 1 | |
| 97 | " | " | " | $H_3\overset{\oplus}{N}(-CH_2)_2-O-CH_2-Phenyl$ | 1 | |
| 98 | $CH(CH_3)_2$ | " | " | $H_3\overset{\oplus}{N}-CH_2-COOH$ | 1 | |
| 99 | " | " | " | $H_3\overset{\oplus}{N}-NH-CONH_2$ | 2 | |
| 100 | " | " | " | $CH_3-\overset{\oplus}{N}H-CH(CH_3)-CH(OH)-Phenyl$ | 1 | |
| 101 | " | " | " | $H_3\overset{\oplus}{N}-C(CH_3)_3$ | 1 | |
| 102 | " | " | " | 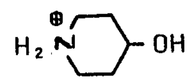 | 1 | |
| 103 | " | " | " | 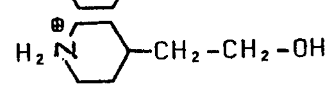 | 1 | |
| 104 | " | " | " | $H\overset{\oplus}{N}(-CH_2-CH(OH)-CH_3)_3$ | 1 | |

Fortsetzung Tabelle 1

| Beisp. Nr. | $R^1$ | $R^2$ | $R^3/n$ | Y | herg. n. Bsp. | Fp [°C] |
|---|---|---|---|---|---|---|
| 105 | $CH_3$ | $CH(CH_3)_2$ | -/0 | $\overset{\oplus}{H}N(-CH_2-CH_2-OH)_3$ | 1 | Harz |
| 106 | $C_2H_5$ | $C_2H_5$ | " | $H_3\overset{\oplus}{N}-C(CH_3)_2-CH_2OH$ | 1 | 152-8 |
| 107 | " | " | " | $(CH_3)_2\overset{\oplus}{N}H-CH_2-CH_2-CN$ | 1 | 100-3 |
| 108 | " | " | " | $[H(H_2N-\overset{NH}{\overset{\|}{C}}-NH(CH_2)_3-CH(NH_2)COOH)]^{\oplus}$ | 1 | 72-5 |
| 109 | " | " | " | $H_3\overset{\oplus}{N}-C(CN)(CH_3)-CH(CH_3)_2$ | 1 | 142-52 |
| 110 | " | " | " | $(CH_3)_3C-\overset{\oplus}{N}H(-CH_2-CH_2-OH)_2$ | 1 | 150-2 |
| 111 | " | " | $4-CH_3/1$ | 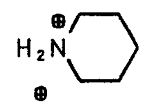 | 1 | 168-72 |
| 112 | " | " | " | $\overset{\oplus}{H}N(-CH_2-CH_2-OH)_3$ | 1 | 67-9 |
| 113 | " | " | $3-CH_3/1$ | 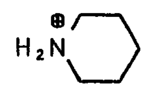 | 1 | 119-22 |
| 114 | " | " | " | $HN(-CH_2-CH_2-OH)_3$ | 1 | 54-8 |

## BIOLOGISCHE BEISPIELE

### 1. Wuchshemmung an Getreide

In Schalenversuchen im Gewächshaus wurden junge Getreidepflanzen (Weizen, Gerste, Roggen) im 3-Blattstadium mit den erfindungsgemäßen Verbindungen in verschiedenen Wirkstoffkonzentrationen (kg/ha) tropfnaß gespritzt.

Nachdem die unbehandelten Kontrollpflanzen eine Wuchshöhe von etwa 55 cm erreicht haben, wird bei allen Pflanzen der Zuwachs gemessen und die Wuchshemmung in % des Zuwachses der Kontrollpflanzen berechnet. Es wird außerdem die phytotoxische Wirkung der Verbindung beobachtet. Die Wuchshemmung wird als prozentualer Wert ermittelt, wobei 100 % den Stillstand des Wachstums und 0 % ein Wachstum entsprechend dem der unbehandelten Kontrollpflanzen bedeuten.

Tabelle I

| Verbindung nach Beispiel Nr. | Anwendungskonzentr. (kg/ha) | Wuchshemmung in % bei Weizen | Gerste | Roggen | Phytotox. Wirkung |
|---|---|---|---|---|---|
| 1 | 2,5 | 22 | 40 | 29 | keine |
| | 1,25 | 17 | 21 | 20 | Schäden |
| 2 | 2,5 | 22 | 39 | 28 | keine |
| | 1,25 | 17 | 20 | 19 | Schäden |
| 3 | 2,5 | 20 | 37 | 27 | keine |
| | 1,25 | 15 | 17 | 16 | Schäden |
| 4 | 2,5 | 21 | 36 | 26 | keine |
| | 1,25 | 14 | 17 | 17 | Schäden |
| 17 | 2,5 | 22 | 40 | 28 | keine |
| | 1,25 | 17 | 22 | 21 | Schäden |

Tabelle I (Fortsetzung)

| Verbindung nach Beispiel Nr. | Anwendungskonzentr. (kg/ha) | Wuchshemmung in % bei | | | Phytotox. Wirkung |
|---|---|---|---|---|---|
| | | Weizen | Gerste | Roggen | |
| 18 | 2,5 | 21 | 39 | 27 | keine |
| | 1,25 | 16 | 20 | 19 | Schäden |
| 19 | 2,5 | 21 | 39 | 28 | keine |
| | 1,25 | 17 | 21 | 17 | Schäden |
| 21 | 2,5 | 20 | 36 | 27 | keine |
| | 1,25 | 16 | 20 | 19 | Schäden |
| 24 | 2,5 | 21 | 39 | 28 | keine |
| | 1,25 | 17 | 17 | 19 | Schäden |
| 25 | 2,5 | 21 | 38 | 27 | keine |
| | 1,25 | 16 | 17 | 18 | Schäden |
| 27 | 2,5 | 22 | 39 | 28 | keine |
| | 1,25 | 17 | 16 | 20 | Schäden |
| 29 | 2,5 | 21 | 39 | 29 | keine |
| | 1,25 | 16 | 17 | 19 | Schäden |
| 33 | 2,5 | 21 | 38 | 29 | keine |
| | 1,25 | 17 | 18 | 19 | Schäden |
| 34 | 2,5 | 21 | 37 | 29 | keine |
| | 1,25 | 17 | 17 | 18 | Schäden |
| 40 | 2,5 | 20 | 36 | 27 | keine |
| | 1,25 | 16 | 15 | 18 | Schäden |
| 42 | 2,5 | 21 | 38 | 28 | keine |
| | 1,25 | 17 | 17 | 19 | Schäden |
| 49 | 2,5 | 21 | 39 | 29 | keine |
| | 1,25 | 17 | 17 | 20 | Schäden |
| 52 | 2,5 | 21 | 36 | 26 | keine |
| | 1,25 | 15 | 17 | 17 | Schäden |
| 53 | 2,5 | 21 | 39 | 26 | keine |
| | 1,25 | 17 | 19 | 20 | Schäden |
| 54 | 2,5 | 21 | 39 | 29 | keine |
| | 1,25 | 17 | 16 | 20 | Schäden |
| 58 | 2,5 | 20 | 36 | 27 | keine |
| | 1,25 | 15 | 16 | 19 | Schäden |

Tabelle I (Fortsetzung)

| Verbindung nach Beispiel Nr. | Anwendungskonzentr. (kg/ha) | Wuchshemmung in % bei | | | Phytotox. Wirkung |
|---|---|---|---|---|---|
| | | Weizen | Gerste | Roggen | |
| 59 | 2,5 | 21 | 38 | 28 | keine |
| | 1,25 | 17 | 18 | 20 | Schäden |
| 64 | 2,5 | 21 | 38 | 27 | keine |
| | 1,25 | 17 | 19 | 18 | Schäden |
| 67 | 2,5 | 21 | 36 | 28 | keine |
| | 1,25 | 17 | 18 | 19 | Schäden |
| 68 | 2,5 | 20 | 37 | 26 | keine |
| | 1,25 | 15 | 16 | 17 | Schäden |
| 73 | 2,5 | 20 | 37 | 29 | keine |
| | 1,25 | 15 | 16 | 18 | Schäden |
| 75 | 2,5 | 21 | 38 | 27 | keine |
| | 1,25 | 16 | 17 | 18 | Schäden |
| 77 | 2,5 | 22 | 37 | 27 | keine |
| | 1,25 | 16 | 19 | 18 | Schäden |
| 79 | 2,5 | 21 | 37 | 29 | keine |
| | 1,25 | 17 | 19 | 21 | Schäden |
| 81 | 2,5 | 22 | 35 | 27 | keine |
| | 1,25 | 16 | 18 | 18 | Schäden |
| 82 | 2,5 | 21 | 35 | 27 | keine |
| | 1,25 | 16 | 14 | 18 | Schäden |
| 88 | 2,5 | 21 | 36 | 29 | keine |
| | 1,25 | 16 | 21 | 16 | Schäden |
| 90 | 2,5 | 21 | 39 | 29 | keine |
| | 1,25 | 17 | 17 | 18 | Schäden |
| 105 | 2,5 | 21 | 36 | 26 | keine |
| | 1,25 | 15 | 18 | 17 | Schäden |
| 108 | 2,5 | 21 | 35 | 27 | keine |
| | 1,25 | 16 | 16 | 19 | Schäden |
| 109 | 2,5 | 24 | 41 | 26 | keine |
| | 1,25 | 19 | 21 | 19 | Schäden |
| 111 | 2,5 | 21 | 37 | 25 | keine |
| | 1,25 | 17 | 21 | 17 | Schäden |

Tabelle I (Fortsetzung)

| Verbindung nach Beispiel Nr. | Anwendungskonzentr. (kg/ha) | Wuchshemmung in % bei | | | Phytotox. Wirkung |
|---|---|---|---|---|---|
| | | Weizen | Gerste | Roggen | |
| 114 | 2,5 | 22 | 36 | 25 | keine |
| | 1,25 | 17 | 18 | 16 | Schäden |
| CCC | 2,5 | 27 | 8 | 10 | keine |
| | 1,25 | 23 | 0 | 0 | Schäden |

= 2-Chlorethyltrimethylammoniumchlorid

2. Wuchshemmung in Wasserreis

Reispflanzen werden in Kleinparzellen (2 m x 2m) angezogen und im Stadium der maximalen Bestockung mit den erfindungsgemäßen Verbindungen behandelt. Die Substanzen werden sowohl durch Spritzung appliziert als auch in das Wasser gegeben.

3 Wochen nach Behandlung wird bei allen Pflanzen der Zuwachs gemessen und die Wuchshemmung in % des Zuwachses der Kontrollpflanzen berechnet. Es wird außerdem auf eine mögliche phytotoxische Wirkung der Verbindungen geachtet.

Die Wuchshemmung wird als prozentualer Wert ermittelt, wobei 100 % den Stillstand des Wachstums und 0 % ein Wachstum entsprechend dem der unbehandelten Kontrollpflanzen bedeuten.

Tabelle II

| Verbindung nach Bsp.Nr. | Anwendungskonz. kg/ha | Wuchshemmung (%) | Phytotox. Wirkung |
|---|---|---|---|
| 1 | 2,5<br>1,25<br>0,62 | 25<br>21<br>19 | keine<br>Schäden |
| 2 | 2,5<br>1,25<br>0,62 | 26<br>20<br>18 | keine<br>Schäden |

Tabelle II (Fortsetzung)

| Verbindung nach Bsp.Nr. | Anwendungs-konz. kg/ha | Wuchshemmung (%) | Phytotox. Wirkung |
|---|---|---|---|
| 3 | 2,5<br>1,25<br>0,62 | 27<br>20<br>16 | keine Schäden |
| 4 | 2,5<br>1,25<br>0,62 | 30<br>22<br>19 | keine Schäden |
| 17 | 2,5<br>1,25<br>0,62 | 29<br>21<br>17 | keine Schäden |
| 18 | 2,5<br>1,25<br>0,62 | 27<br>19<br>17 | keine Schäden |
| 19 | 2,5<br>1,25<br>0,62 | 30<br>22<br>18 | keine Schäden |
| 21 | 2,5<br>1,25<br>0,62 | 31<br>21<br>17 | keine Schäden |
| 24 | 2,5<br>1,25<br>0,62 | 31<br>20<br>16 | keine Schäden |
| 25 | 2,5<br>1,25<br>0,62 | 26<br>20<br>16 | keine Schäden |
| 27 | 2,5<br>1,25<br>0,62 | 27<br>19<br>16 | keine Schäden |
| 29 | 2,5<br>1,25<br>0,62 | 26<br>20<br>16 | keine Schäden |
| 33 | 2,5<br>1,25<br>0,62 | 27<br>21<br>18 | keine Schäden |

Tabelle II (Fortsetzung)

| Verbindung nach Bsp.Nr. | Anwendungs-konz. kg/ha | Wuchshemmung (%) | Phytotox. Wirkung |
|---|---|---|---|
| 34 | 2,5<br>1,25<br>0,62 | 30<br>22<br>19 | keine Schäden |
| 40 | 2,5<br>1,25<br>0,62 | 27<br>23<br>19 | keine Schäden |
| 42 | 2,5<br>1,25<br>0,62 | 31<br>22<br>19 | keine Schäden |
| 49 | 2,5<br>1,25<br>0,62 | 32<br>25<br>18 | keine Schäden |
| 52 | 2,5<br>1,25<br>0,62 | 31<br>22<br>17 | keine Schäden |
| 53 | 2,5<br>1,25<br>0,62 | 33<br>25<br>19 | keine Schäden |
| 54 | 2,5<br>1,25<br>0,62 | 29<br>24<br>17 | keine Schäden |
| 58 | 2,5<br>1,25<br>0,62 | 29<br>23<br>18 | keine Schäden |
| 59 | 2,5<br>1,25<br>0,62 | 27<br>21<br>16 | keine Schäden |
| 64 | 2,5<br>1,25<br>0,62 | 29<br>21<br>16 | keine Schäden |
| 67 | 2,5<br>1,25<br>0,62 | 30<br>22<br>18 | keine Schäden |

Tabelle II (Fortsetzung)

| Verbindung nach Bsp.Nr. | Anwendungs-konz. kg/ha | Wuchshemmung (%) | Phytotox. Wirkung |
|---|---|---|---|
| 68 | 2,5<br>1,25<br>0,62 | 29<br>25<br>17 | keine Schäden |
| 73 | 2,5<br>1,25<br>0,62 | 29<br>24<br>16 | keine Schäden |
| 75 | 2,5<br>1,25<br>0,62 | 30<br>25<br>17 | keine Schäden |
| 77 | 2,5<br>1,25<br>0,62 | 29<br>24<br>16 | keine Schäden |
| 79 | 2,5<br>1,25<br>0,62 | 30<br>24<br>18 | keine Schäden |
| 81 | 2,5<br>1,25<br>0,62 | 29<br>23<br>18 | keine Schäden |
| 82 | 2,5<br>1,25<br>0,62 | 29<br>21<br>16 | keine Schäden |
| 88 | 2,5<br>1,25<br>0,62 | 27<br>19<br>16 | keine Schäden |
| 90 | 2,5<br>1,25<br>0,62 | 27<br>20<br>16 | keine Schäden |
| 105 | 2,5<br>1,25<br>0,62 | 26<br>19<br>16 | keine Schäden |
| 108 | 2,5<br>1,25<br>0,62 | 27<br>20<br>16 | keine Schäden |

Tabelle II (Fortsetzung)

| Verbindung nach Bsp.Nr. | Anwendungs-konz. kg/ha | Wuchshemmung (%) | Phytotox. Wirkung |
|---|---|---|---|
| 109 | 2,5 | 27 | keine Schäden |
| | 1,25 | 21 | |
| | 0,62 | 17 | |
| 111 | 2,5 | 31 | keine Schäden |
| | 1,25 | 25 | |
| | 0,62 | 19 | |
| 114 | 2,5 | 31 | keine Schäden |
| | 1,25 | 24 | |
| | 0,62 | 18 | |

3. Wuchshemmung an Sojabohnen

Ca. 10 cm große Sojabohnen werden mit den Wirkstoffzubereitungen tropfnaß bespritzt. Nach 3 Wochen wird bonitiert.

Die Wuchshemmung wird als prozentualer Wert ermittelt, wobei 100 % den Stillstand des Wachstums und 0 % ein Wachstum entsprechend dem der unbehandelten Kontrollpflanzen bedeuten.

Tabelle III

| Verbindung nach Bsp.Nr. | Anwendungs-konz. kg/ha | Wuchshemmung (%) | Phytotox. Wirkung |
|---|---|---|---|
| 1 | 2,5 | 25 | keine Schäden |
| 2 | 2,5 | 24 | keine Schäden |
| 3 | 2,5 | 26 | keine Schäden |
| 4 | 2,5 | 27 | keine Schäden |
| 17 | 2,5 | 24 | keine Schäden |
| 18 | 2,5 | 23 | keine Schäden |

Tabelle III (Fortsetzung)

| Verbindung nach Bsp.Nr. | Anwendungs- konz. kg/ha | Wuchshemmung (%) | Phytotox. Wirkung |
|---|---|---|---|
| 19 | 2,5 | 28 | keine Schäden |
| 21 | 2,5 | 27 | keine Schäden |
| 24 | 2,5 | 22 | keine Schäden |
| 25 | 2,5 | 22 | keine Schäden |
| 27 | 2,5 | 24 | keine Schäden |
| 29 | 2,5 | 19 | keine Schäden |
| 33 | 2,5 | 26 | keine Schäden |
| 34 | 2,5 | 24 | keine Schäden |
| 40 | 2,5 | 21 | keine Schäden |
| 42 | 2,5 | 26 | keine Schäden |
| 49 | 2,5 | 29 | keine Schäden |
| 52 | 2,5 | 27 | keine Schäden |
| 53 | 2,5 | 22 | keine Schäden |
| 54 | 2,5 | 25 | keine Schäden |
| 58 | 2,5 | 26 | keine Schäden |
| 59 | 2,5 | 23 | keine Schäden |
| 64 | 2,5 | 24 | keine Schäden |
| 67 | 2,5 | 22 | keine Schäden |
| 68 | 2,5 | 25 | keine Schäden |
| 73 | 2,5 | 27 | keine Schäden |
| 75 | 2,5 | 31 | keine Schäden |
| 77 | 2,5 | 25 | keine Schäden |
| 79 | 2,5 | 21 | keine Schäden |
| 81 | 2,5 | 24 | keine Schäden |
| 82 | 2,5 | 27 | keine Schäden |
| 88 | 2,5 | 27 | keine Schäden |
| 90 | 2,5 | 22 | keine Schäden |
| 105 | 2,5 | 21 | keine Schäden |
| 108 | 2,5 | 23 | keine Schäden |
| 109 | 2,5 | 27 | keine Schäden |
| 111 | 2,5 | 23 | keine Schäden |
| 114 | 2,5 | 25 | keine Schäden |
| CCC | 2,5 | 10 | keine Schäden |

4. Synergistische Effekte

Im Freiland wird ein Stück Feld, auf dem verschiedene Getreidearten angezogen sind, für einen Kleinparzellenversuch mit den erfindungsgemäßen Verbindungen ausgewählt. Dann werden die Wachstumsregulatoren im Nachauflaufverfahren in verschiedenen Entwicklungsstadien mit einem Wasseraufwandvolumen von 400 l/ha auf 10 $m^2$ große Versuchsparzellen appliziert. Es werden jeweils 4 Wiederholungen behandelt. Bonitiert wird im 14-tägigen Abstand nach Applikation und zwar Phytotoxizität, Wuchshöhe, Entwicklungsverlauf und Ertragskomponenten. Hierbei zeigt sich nun, daß Mischungen der Wachstumsregulatoren der Formel (I) in Kombination mit einem Wirkstoff der Formel (IV), (V) oder (XII) hervorragende synergistische Effekte zeigen. Es wird eine wesentlich bessere Halmverkürzung erzielt als auf Grund der Einzelwirkung der Komponenten zu erwarten wäre (additive Wirkung). Diese synergistische Wirkung tritt in einem breiten Bereich der pflanzlichen Entwicklung auf.

PATENTANSPRÜCHE: HOE 84/F 280

1. Salze der im Phenylring substituierten 1-Phenyl-imidazol-5-carbonsäuren, dadurch gekennzeichnet, daß in der allgemeinen Formel (I)

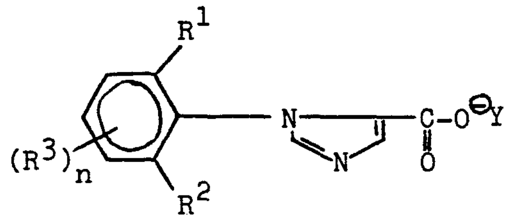

(I)

die Symbole folgende Bedeutung haben:

Y 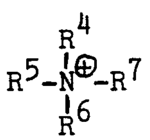 , 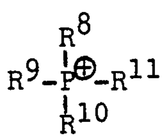 , 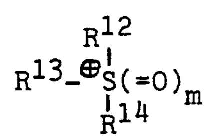 ,

das Argininiumion oder das Guanidiniumion

n 0, 1, 2 oder 3;

m 0 oder 1;

$R^1,R^2$ unabhängig voneinander $(C_1-C_4)$Alkyl,

$R^3$ $(C_1-C_4)$Alkyl, $(C_1-C_4)$Alkoxy oder Halogen, wobei bei n > 1 die Substituenten auch unterschiedlich sein können;

$R^4,R^5,R^6$ unabhängig voneinander H; unsubstituiertes oder bis zu dreifach, vorzugsweise einfach durch Halogen, $(C_1-C_{12})$Alkoxy, vorzugsweise $(C_1-C_6)$-Alkoxy, $[(C_1-C_6)$Alkoxy$]-(C_2-C_4)$alkoxy, Cyclo-$(C_3-C_7)$alkyl, Bicyclo$(C_7-C_{10})$alkyl, Benzyloxy, Halogen-benzyloxy, Methyl-benzyloxy, Benzyloxy-$(C_2-C_4)$alkoxy, Phenyl, Halogen-phenyl, Methyl-phenyl, Cyano, Hydroxyl, Formyl, $(C_1-C_4)$Alkylcarbonyl, Carboxyl, $(C_1-C_6)$-Alkoxycarbonyl, Carbamoyl, Phosphinyl, Phosphonyl, $(C_1-C_4)$-Alkylamino-phosphonyl, Di$(C_1-C_4)$alkylamino-phosphonyl, Amino, $(C_1-C_6)$Alkylamino, Di$(C_1-C_6)$alkylamino, Amino-$(C_1-C_4)$alkylamino, $(C_1-C_6)$Alkylthio, Phenylthio, Phenoxy, Furyl, Tetrahydrofuryl, Imidazolyl oder Triazolyl substituiertes $(C_1-C_{18})$Alkyl; unsubstituiertes oder durch Halogen oder Phenyl substituiertes $(C_3-C_6)$Alkenyl; $(C_3-C_6)$Alkinyl; unsubstituiertes oder durch $(C_1-C_4)$Alkyl oder Halogen substituiertes Cyclo$(C_3-C_8)$alkyl; Cyclo$(C_5C_6)$alkenyl; unsubstituiertes oder bis zu dreifach durch Halogen, Nitro, Cyano, $(C_1-C_4)$Alkyl, $(C_1-C_4)$Alkoxycarbonyl oder $(C_1-C_4)$Alkoxy substituiertes Phenyl;
oder
$R^4$ und $R^5$ bilden gemeinsam mit dem N eine Cyclo$(C_5-C_8)$alkylgruppe, bei der zusätzlich zu dem einen N noch bis zu 2 C durch N, S und/oder O ersetzt sein können und die unsubstituiert oder durch $(C_1-C_4)$Alkyl, Halogen, Phenyl, Benzyl, Oxo, Amino, $(C_1-C_4)$Alkylamino, Di$(C_1-C_4)$alkylamino, Amino$(C_1-C_4)$-alkyl, Hydroxyl, Hydroxy-$(C_1-C_4)$alkyl, $(C_1-C_4)$Alkoxy substituiert ist;

$R^7$ H; unsubstituiertes oder durch Phenyl, Halogenphenyl oder Methyl-phenyl substituiertes $(C_1-C_{12})$Alkyl; oder

bei $R^4$, $R^5$ und $R^6$ = H Amino, $(C_1-C_4)$Alkylamino, Di$(C_1-C_4)$alkylamino, Benzylamino, Anilino, Formylamino, $(C_1-C_4)$Alkylcarbonylamino, Benzoylamino, Hydroxyl, $(C_1-C_6)$Alkoxy oder $NH-C(NH_2)=Z$ mit Z=O,S oder NH;

ausgenommen sind Verbindungen, bei denen $R^4$, $R^5$, $R^6$ und $R^7$ = H sind;

$R^8, R^9, R^{10}, R^{11}$ unabhängig voneinander $(C_1-C_{18})$Alkyl; unsubstituiertes oder durch Halogen, $(C_1-C_4)$Alkyl oder $(C_1-C_4)$Alkoxy substituiertes Phenyl; oder unsubstituiertes oder durch Halogen, $(C_1-C_4)$Alkyl oder $(C_1-C_4)$Alkoxy substituiertes Benzyl; und

$R^{12}, R^{13}, R^{14}$ unabhängig voneinander $(C_1-C_6)$Alkyl, Phenyl, Halogen-phenyl, Methyl-phenyl, Benzyl, Halogen-benzyl oder Methyl-benzyl.

2. Herstellung der Salze der allgemeinen Formel (I) nach Anspruch 1 aus den freien Carbonsäuren oder Metallsalzen der allgemeinen Formeln (II) und (III) mit $Met^+$ in der Bedeutung eines einfach positiv geladenen Metallkations bzw. eines Kationäquivalents bei einem mehrfach positiv geladenen Metallkation,

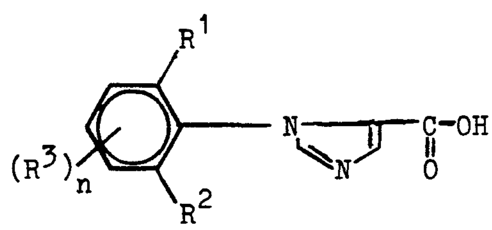

(II)

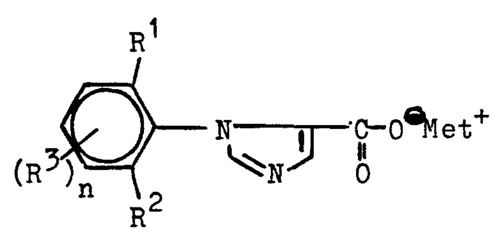

(III)

gekennzeichnet

a) durch Umsetzung von (II) mit den Aminoverbindungen Y' (Y'=Y-$H^+$) oder mit Salzen mit Y als Kation, wobei Y

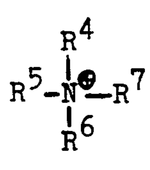

,

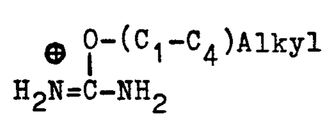

oder

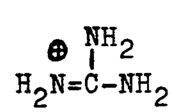

bedeutet und mindestens einer der Reste $R^4$ bis $R^7$=H, oder

b) durch Umsetzung von (II) mit den Verbindungen $YHal^-$ oder $YOH^-$ oder von (III) mit den Verbindungen $YHal^-$, wobei Y

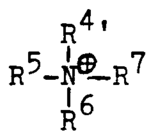 , 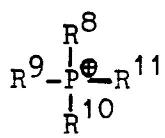 oder 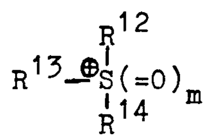 ,

und alle Reste $R^4$ bis $R^7$ ≠ H sind.

3. Das Pflanzenwachstum regulierende Mittel mit einem wirksamen Gehalt an mindestens einer Verbindung der allgemeinen Formel (I) nach Anspruch 1.

4. Verwendung von Verbindungen der allgemeinen Formel (I) nach Anspruch 1 als Wachstumsregulatoren für Pflanzen.

5. Verfahren zur Wachstumsregulierung von Pflanzen, dadurch gekennzeichnet, daß man auf die Pflanzen oder die Anbaufläche eine wirksame Menge mindestens einer Verbindung der allgemeinen Formel (I) nach Anspruch 1 appliziert.

Patentansprüche Österreich:

1. Pflanzenwachstumregulierende Mittel, dadurch gekennzeichnet, daß sie eine Verbindung der allgemeinen Formel (I)

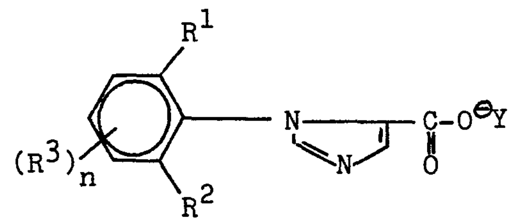

(I)

worin

Y 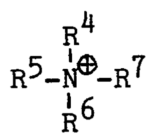 , 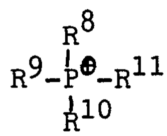 , 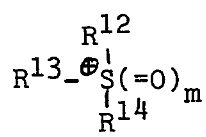 ,

das Argininiumion oder das Guanidiniumion

n 0, 1, 2 oder 3;

m 0 oder 1;

$R^1$,$R^2$ unabhängig voneinander $(C_1-C_4)$Alkyl,

$R^3$ $(C_1-C_4)$Alkyl, $(C_1-C_4)$Alkoxy oder Halogen, wobei bei n $>1$ die Substituenten auch unterschiedlich sein können;

$R^4$,$R^5$,$R^6$ unabhängig voneinander H; unsubstituiertes oder bis zu dreifach, vorzugsweise einfach durch Halogen, $(C_1-C_{12})$Alkoxy, vorzugsweise $(C_1-C_6)$-Alkoxy, $[(C_1-C_6)$Alkoxy$]-(C_2-C_4)$alkoxy, Cyclo-$(C_3-C_7)$alkyl, Bicyclo$(C_7-C_{10})$alkyl, Benzyloxy, Halogen-benzyloxy, Methyl-benzyloxy, Benzyloxy-$(C_2-C_4)$alkoxy, Phenyl, Halogen-phenyl, Methyl-phenyl, Cyano, Hydroxyl, Formyl, $(C_1-C_4)$Alkylcarbonyl, Carboxyl, $(C_1-C_6)$-Alkoxycarbonyl, Carbamoyl, Phosphinyl, Phosphonyl, $(C_1-C_4)$-Alkylamino-phosphonyl, Di$(C_1-C_4)$alkylamino-phosphonyl, Amino, $(C_1-C_6)$Alkylamino, Di$(C_1-C_6)$alkylamino, Amino-$(C_1-C_4)$alkylamino, $(C_1-C_6)$Alkylthio, Phenylthio, Phenoxy, Furyl, Tetrahydrofuryl, Imidazolyl oder Triazolyl substituiertes $(C_1-C_{18})$Alkyl; unsubstituiertes oder durch Halogen oder Phenyl substituiertes $(C_3-C_6)$Alkenyl; $(C_3-C_6)$Alkinyl; unsubstituiertes oder durch $(C_1-C_4)$Alkyl oder Halogen substituiertes Cyclo$(C_3-C_8)$alkyl; Cyclo$(C_5C_6)$alkenyl; unsubstituiertes oder bis zu dreifach durch Halogen, Nitro, Cyano, $(C_1-C_4)$Alkyl, $(C_1-C_4)$Alkoxycarbonyl oder $(C_1-C_4)$Alkoxy substituiertes Phenyl;

oder

$R^4$ und $R^5$ bilden gemeinsam mit dem N eine Cyclo$(C_5-C_8)$alkylgruppe, bei der zusätzlich zu dem einen N noch bis zu 2 C durch N, S und/oder O ersetzt sein können und die unsubstituiert oder durch $(C_1-C_4)$Alkyl, Halogen, Phenyl, Benzyl, Oxo, Amino, $(C_1-C_4)$Alkylamino, Di$(C_1-C_4)$alkylamino, Amino$(C_1-C_4)$-alkyl, Hydroxyl, Hydroxy-$(C_1-C_4)$alkyl, $(C_1-C_4)$Alkoxy substituiert ist;

$R^7$ H; unsubstituiertes oder durch Phenyl, Halogenphenyl oder Methyl-phenyl substituiertes $(C_1-C_{12})$Alkyl; oder

bei $R^4$, $R^5$ und $R^6$ = H Amino, $(C_1-C_4)$Alkylamino, Di-$(C_1-C_4)$alkylamino, Benzylamino, Anilino, Formylamino, $(C_1-C_4)$Alkylcarbonylamino, Benzoylamino, Hydroxyl, $(C_1-C_6)$Alkoxy oder NH-C=Z mit Z=O,S oder NH;

$NH_2$

ausgenommen sind Verbindungen, bei denen $R^4$, $R^5$, $R^6$ und $R^7$ = H sind;

$R^8$, $R^9$, $R^{10}$, $R^{11}$ unabhängig voneinander $(C_1-C_{18})$Alkyl; unsubstituiertes oder durch Halogen, $(C_1-C_4)$Alkyl oder $(C_1-C_4)$Alkoxy substituiertes Phenyl; oder unsubstituiertes oder durch Halogen, $(C_1-C_4)$Alkyl oder $(C_1-C_4)$Alkoxy substituiertes Benzyl; und

$R^{12}$, $R^{13}$, $R^{14}$ unabhängig voneinander $(C_1-C_6)$Alkyl, Phenyl, Halogen-phenyl, Methyl-phenyl, Benzyl, Halogenbenzyl oder Methyl-benzyl, bedeuten,

enthalten.

2. Herstellung der Salze der allgemeinen Formel (I) nach Anspruch 1 aus den freien Carbonsäuren oder Metallsalzen der allgemeinen Formeln (II) und (III) mit $Met^+$ in der Bedeutung eines einfach positiv geladenen Metallkations bzw. eines Kationäquivalents bei einem mehrfach positiv geladenen Metallkation,

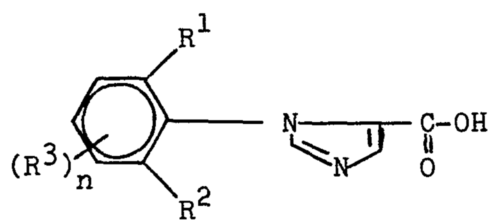

(II)

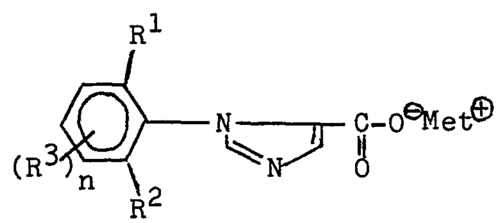

(III)

gekennzeichnet

a) durch Umsetzung von (II) mit den Aminoverbindungen Y' ($Y'=Y-H^+$) oder mit Salzen mit Y als Kation, wobei Y

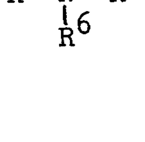

,



oder

bedeutet und mindestens einer der Reste $R^4$ bis $R^7$=H, oder

b) durch Umsetzung von (II) mit den Verbindungen Y $Hal^-$ oder $YOH^-$ oder von (III) mit den Verbindungen $YHal^-$, wobei Y

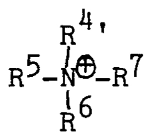 , 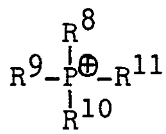 oder 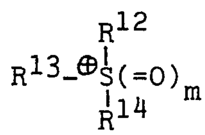 ,

und alle Reste $R^4$ bis $R^7$ ≠ H sind.

3. Verwendung von Verbindungen der allgemeinen Formel (I) nach Anspruch 1 als Wachstumsregulatoren für Pflanzen.

4. Verfahren zur Wachstumsregulierung von Pflanzen, dadurch gekennzeichnet, daß man auf die Pflanzen oder die Anbaufläche eine wirksame Menge mindestens einer Verbindung der allgemeinen Formel (I) nach Anspruch 1 appliziert.

0182246

Europäisches Patentamt

# EUROPÄISCHER RECHERCHENBERICHT

Nummer der Anmeldung

EP 85114339.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 732 531 (HOECHST)<br>* Ansprüche 1,4 *<br>-- | 1,4,5 | C 07 D 233/92<br>A 01 N 43/50 |
| A | US - A - 4 292 431 (KIM)<br>* Formel II *<br>-- | 1 | |
| A | CHEMICAL ABSTRACTS, Vol. 94, No. 5, 2. Februar 1981, Columbus, Ohio, USA<br>BELGODERE, ELENA; BOSSIO, R.; PARRINI, V.; PEPINO, R. "Imidazole derivatives with potential biological activity." Seite 563, Spalte 1, Zusammenfassung Nr. 30642u<br>& Arzneim.-Forsch. 1980, 30(7), 1051-6<br>---- | 1 | RECHERCHIERTE SACHGEBIETE (Int. Cl.4)<br>C 07 D 233/00 |

Der vorliegende Recherchenbericht wurde für alle Patentansprüche erstellt.

| Recherchenort | Abschlußdatum der Recherche | Prüfer |
|---|---|---|
| WIEN | 17-02-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82